# EUROPEAN PATENT APPLICATION

(11) **EP 3 306 335 A1**
(43) Date of publication of application: **11.04.2018**
(21) Application number: 16192739.7
(22) Date of filing: 07.10.2016
(51) Int. Cl.: G01R 33/48, A61N 5/10, G01R 33/56, A61B 5/055

(54) **APPARATUS AND METHOD FOR LOCALIZING THE BRAGG PEAK OF A HADRON BEAM TRAVERSING A TARGET TISSUE BY MAGNETIC RESONANCE IMAGING**

(71) Applicant: ION BEAM APPLICATIONS S.A., 1348 Louvain-la-Neuve (BE)
(72) Inventor: PRIEELS, Damien, 1348 Louvain-la-Neuve (BE); HENROTIN, Sébastien, 1348 Louvain-la-Neuve (BE); VAN DER KRAAIJ, Erik, 1348 Louvain-la-Neuve (BE); BRUSASCO, Caterina, 1348 Louvain-la-Neuve (BE)
(74) Representative: Connor, Marco Tom

(57) **Abstract**

The present invention concerns a method and a medical apparatus for visualizing a hadron beam traversing an organic body, said method comprising capturing a magnetic resonance image wherein a volume of irradiated excitable atoms A1, surrounding a hadron beam and whose magnetic susceptibility has been modified by said hadron beam is captured as a hyposignal. A hyposignal is obtained by saturating the spins of the irradiated excitable atoms A1, before capturing a MR image based on the excitation of the excitable atoms A0 which are not affected by the hadron beam.

## Description

### Field of the invention

The present invention relates to a medical apparatus comprising a charged hadron therapy device coupled to a magnetic resonance imaging device (MRI) adapted for visualizing *in situ* the position of the Bragg peak of a hadron beam traversing a target tissue relative to the position of a target spot in said target tissue. The *in situ* localization of the actual position of the Bragg peak relative to the target spot, immediately before a hadron therapy session starts is highly useful for validating the planned position of the Bragg peak of the hadron beam determined during an earlier established treatment plan for treating said target spot. If a discrepancy appears between the planned and actual positions of the Bragg peak of the hadron beam, the present invention may allow the correction of the initial energy, **E1,** of the hadron beam required for positioning the Bragg peak over the target spot. The hadron therapy session needs not be cancelled and may thus proceed with corrected parameters.

### Description of prior art

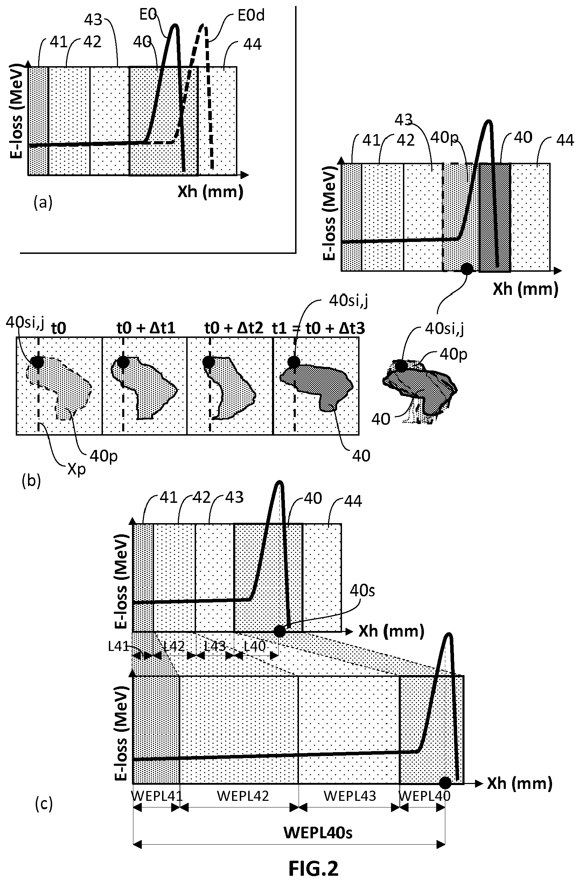

Hadron therapy (for example, proton therapy) for treating a patient has been known for a couple of decades with the prospect of several advantages over conventional radiotherapy. These advantages are due to the physical nature of hadrons. A photon beam in conventional radiotherapy releases its energy according to a decreasing exponential curve as a function of the distance of tissue traversed by the photon beam. By contrast and such as illustrated in Figure 2, a hadron beam first releases a small fraction of its energy as it penetrates tissues **41-43,** forming a plateau then, as the hadron path is prolonged, the whole energy is released locally following a steep increase to a peak and a fall-off at the end of the range of the beam. The peak is called Bragg peak and corresponds to the maximum of the Bragg curve illustrated in Figure 2(c). Consequently, a hadron beam can deliver a high dose of hadrons at a precise location within a target tissue **40,** thus preserving the surrounding healthy tissues **41-44.** As illustrated in Figure 2(a), the advantage of hadron therapy allowing the delivery of high doses of hadron at a precise location is also one of its weaknesses, because if the position, **BP0**, of the Bragg peak of a hadron beam is offset relative to the target tissues **40,** high doses of hadrons may be delivered to adjacent tissues **43, 44** which are healthy (compare solid line, **E0,** and dashed line, **E0d,** of the curves of energy loss, Eₗₒₛₛ, with respect to the distance, **xh,** travelled by the hadron beam within tissues and measured along the beam path, **Xp,** in Figure 2(a)). For this reason, the determination of the relative position of the Bragg peak with respect to the position of the target tissue is crucial to properly implement hadron therapy to a patient.

In practice, hadron therapy usually requires the establishment of a treatment plan before any treatment can start. During this treatment plan, a computer tomography scan (CT scan) of the patient and target tissues is usually performed. The CT scan is used to characterize the target tissue **40** and the surrounding tissues **41-43** to be traversed by a treatment hadron beam **1h** for the treatment of a patient. The characterization yields a 3D representation of the volume comprising the target tissue, and a treatment plan system determines a range-dose calculated based on the nature of the tissues **41-43** traversed by the hadron beam.

This characterization permits computation of a water equivalent path length (WEPL), which is used for determining the initial energy, **Ek,** of the treatment hadron beam required for delivering a prescribed dose of hadrons to a target spot **40s,** wherein k = 0 or 1 depending on the stage when said initial energy was determined. Figure 2(c) illustrates the conversion of the physical distances travelled by a hadron beam traversing different tissues into corresponding WEPL's. The WEPL of a hadron beam travelling a given distance through a given tissue is the equivalent distance said hadron beam would travel in water. As illustrated in Figure 2(c) if, as is usually the case, healthy tissues **41-43** of different natures and thicknesses separate a target tissue from the outer surface of the skin of a patient, the WEPL of a target spot is calculated taking into account the water corresponding path lengths of each tissue in series until the target spot is reached. With a value of the equivalent path length of a hadron beam traveling in water, the initial energy, **Ek,** required for positioning the Bragg peak at the WEPL of the target spot can easily be computed and corresponds to the initial energy, **Ek,** required for positioning the Bragg peak at the target spot within the target tissue.

The treatment plan can then be executed during a treatment phase including one or more treatment sessions during which doses of hadrons are deposited onto the target tissue. The position of the Bragg peak of a hadron beam with respect to the target spots of a target tissue, however, suffers of a number of uncertainties including:
- the variations of the patient position, on the one hand, during a hadron therapy session and, on the other hand, between the establishment of the treatment plan and the hadron therapy session;
- the variations of the size and/or of the position of the target tissue (see Figure 2(b)) and/or of the healthy tissues **41-43** positioned upstream from the target tissue with respect to the hadron beam.
- the range calculation from CT scans is limited by the quality of the CT images. Another limitation is linked to the fact that CT scans use the attenuation of X-rays that have to be converted in hadron attenuation which is non obvious and depends on the chemical composition of the tissues traversed.

The uncertainty on the position of the patient and, in particular, of the target tissue is critical for obvious reasons. Even with an accurate characterization by CT scan, the actual position of a target tissue during a treatment session remains difficult to ascertain for the following reasons:
- first, during an irradiation session, the position of a target tissue can change because of anatomical processes such as breathing, digestion, or heartbeats of the patient. Anatomical processes can also cause gases or fluids appearing or disappearing from the beam path, **Xp,** of a hadron beam.
- second, treatment plans are usually determined several days or weeks before a hadron treatment session starts and treatment of a patient can take several weeks distributed over several treatment sessions. During this time period, the patient can lose or gain weight, therefore modifying, sometimes significantly, the volume of tissues such as fats and muscles.

Accordingly, the size of the target tissue can change (e.g. a tumour may have grown, receded, or changed position or geometry). Figure 2(b) shows an example of evolution of the size and position of a target tissue **40** between the time, **t0**, of the establishment of the treatment plan and the times, **t0** + **Δt1, t0** + **Δt2, t1** = **t0** + **Δt3,** of treatment sessions. The treatment plan and last treatment session may be separated by several days or weeks. The treatment plan established at time, **t0**, may therefore comprise irradiation of a target spot **40si,j** (black spot in Figure 2(b)) which belonged to the target tissue **40p** at said time, **t0**. Because the target tissue **40p** may have moved or changed shape during the period, **Δt3,** said target spot **40si,j** may not belong to the target tissue **40** anymore at the time, **t0** + **Δt3,** of the treatment session and be located in a healthy tissue instead. Consequently, irradiating said target spot would hit and possibly harm healthy tissues **43** instead of target tissues **40.**

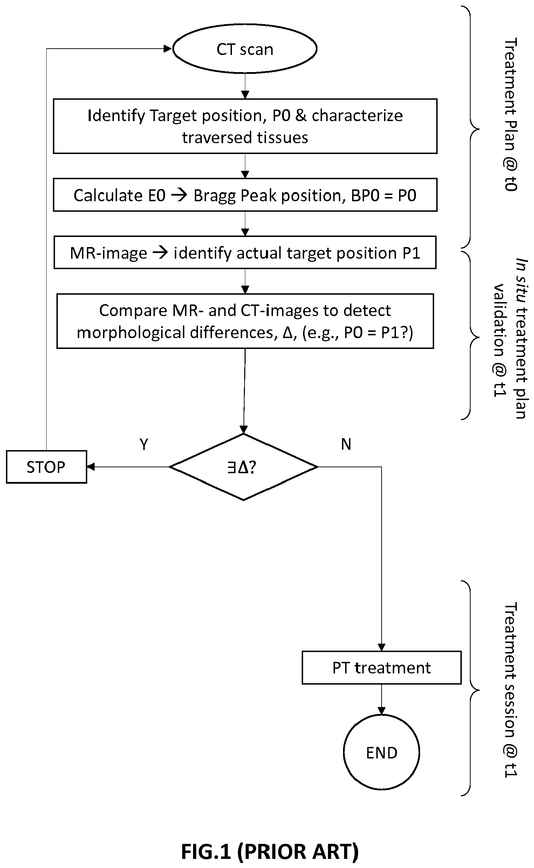

The use of a magnetic resonance imaging device (MRI) coupled to a hadron therapy device has been proposed in the art for identifying any variation of the size and /or the position of a target tissue. For example, US patent 8427148 describes a system comprising a hadron therapy device coupled to a MRI. Said system can acquire images of the patient during a hadron therapy session and can compare these images with CT scan images of the treatment plan. Present Figure 1 illustrates an example of a known flowchart of a hadron therapy session using a hadron therapy device coupled to an MRI. A treatment plan is established including the characterization of the target tissue **40s** and surrounding tissues **41-43.** This step is traditionally performed with a CT scan analysis and allows the determination of the position, **P0**, and morphology of a target tissue, the best trajectories or beam paths, **Xp,** of hadron beams for the hadron treatment of the target tissue, and characterization of the sizes and natures of the tissues traversed by a hadron beam following said beam paths, **Xp,** to determine WEPL40s's of target spots of the said target tissue. The initial energies, **Ek,** of the hadron beams required for matching the corresponding positions, **BP0**, of the Bragg peaks of the hadron beams to the position, **P0**, of the target tissue can thus be calculated. This completes the establishment of a treatment plan.

A hadron therapy session follows the establishment of the treatment plan. With an MRI coupled to a hadron therapy device, it is possible to capture a magnetic resonance (MR) image of a volume, **Vp,** including the target tissue and surrounding tissues to be traversed by a hadron beam. The MR image can then be compared with CT scan images to assess whether any morphological differences, Δ, can be detected in the imaged tissues between the time the CT scans were performed (= **t0** in Figure 2(b)) and the time of the hadron therapy session **(t1= t0** + **Δt3** in Figure 2(b)). If no substantial difference in morphology affecting the treatment session can be detected, then the hadron therapy session proceeds as planned in the treatment plan. If, on the other hand, some differences are detected that could influence the relative position of the target tissue with respect to the planned hadron beams and their respective Bragg peaks, the hadron therapy session is interrupted and a new treatment plan must be established. This technique proposed in the art is greatly advantageous because it can prevent carrying out a hadron therapy session based on a treatment plan which has become obsolete, thus preventing healthy tissues from being irradiated instead of the target tissue.

The magnetic resonance (MR) images provide high contrast of soft tissue traversed by a hadron beam but, to date, have not been suitable for visualizing the hadron beam itself, let alone the position of the Bragg peak because:
- MRI measures the density of hydrogen atoms in tissues, but to date does not yield any identifiable information on the hadron stopping power ratio. The conversion from density of hydrogen atoms to the hadron stopping power ratio suffers from uncertainties similar to and yet less understood than those of the conversion from X-rays in CT scan.
- Due to the different techniques used in CT scan and in MRI, the comparison between the images from CT scan and the images from MRI is not obvious and can suffer from uncertainties.

In conclusion, whilst in hadron therapy, an accurate determination of the position of the Bragg peak relative to the portion of a target tissue is crucial because errors on this position may lead to the irradiation of healthy tissues rather than irradiation of target tissues, no satisfactory solution for determining the relative positions of the Bragg peak and target tissues is available to date. Apparatuses combining a hadron therapy device and a MRI proposed in the art allow *in situ* acquisition of images during a treatment session thus giving information related to the actual position of the target tissue. Said images are, however, not sufficient for ensuring the exact determination of the position of the Bragg peak of a hadron beam and of where it stands relative to the target tissue. There therefore remains a need for a hadron therapy device combined to MRI allowing a better determination of the position of the Bragg peak relative to the position of a target tissue.

### Summary of the invention

The present invention is defined in the appended independent claims. Preferred embodiments are defined in the dependent claims. In particular, the present invention concerns a method for visualizing a hadron beam traversing an organic body, said method comprising:
(a) determining:
   - the Larmor rest frequency, **fLm**,**0**, of an excitable atom present in a target tissue m of a subject of interest, wherein **m** = 40, corresponding to a target tissue, and exposed to a uniform magnetic field, B0, and
   - the Larmor irradiated frequency, **fLm**,**1**, of irradiated excitable atoms defined as the excitable atoms present in the same tissue **m,** and exposed to the effects of a hadron beam of initial energy, **Ek,** with **k** = 0 or 1, traversing said target tissue positioned in the same magnetic field, **B0,** and
(b) calculating the frequency shift, **ΔfLm** = **|fLm,1 - fLm,01,** in the target tissue, with **m** = 40;
(c) providing a magnetic resonance imaging device (MRI) for acquiring magnetic resonance data within an imaging volume, **Vp,** including the target tissue, positioned in the uniform main magnetic field, **B0;**
(d) providing a hadron source adapted for directing a hadron beam having an initial energy, **E0,** along a beam path, **Xp,** intersecting the target tissue in the imaging volume;
(e) acquiring magnetic resonance data from the imaging volume, by at least an excitation step (MRe), the excitation step comprising:
   - an A1-saturation step, comprising creating **n** bursts of a saturating electromagnetic field, **B1-sat,** wherein **n** is an integer greater than 0, which oscillates at a frequency range, **[fL1],** of band width, **b1** < **2·ΔfL40,** and centred on the Larmor irradiated frequency, **fL40,1,** and excluding the Larmor rest frequency, **fL40,0,** such as to bring the nuclei of the irradiated excitable atoms to a saturated state wherein a net polarization vector of the spins is reversed at an angle comprised between 100 and 180° with respect to the net polarization vector of the spins of said nuclei at rest (i.e., absent **B1-sat)** and, after a time, **Δts-e,** following the n^{th} burst **B1-sat:**
   - an AO-excitement step comprising creating **p** bursts of an exciting electromagnetic field, B1-exc, oscillating at a frequency range, **[fL0],** centred on the Larmor rest frequency, **fL40,0,** wherein **p** is an integer greater than 0, such as to bring to an excited state the excitable atoms which are not affected by the hadron beam and which were therefore not brought to a saturated state by the saturating electromagnetic field, **B1-sat,**
(f) directing a hadron beam having the initial energy, **E0,** along a beam path intersecting said target body in a number, **N,** of hadron pulses of pulse periods, **Pbi ,** wherein, **N** is an integer greater than 0;
(g) representing on a display the organic body from the magnetic resonance data acquired by the MRI within the imaging volume, **Vp,**
(h) on the display, visualizing the beam path in the target tissue as a hyposignal, weaker than the signal generated by the excitable atoms (A0) that are not exposed significantly to the effects of the hadron beam.

The present invention is characterized in that, the N hadron pulses overlap with at least 50% of the n bursts of the saturating electromagnetic field, B1-sat during the A1-stauration step. The N hadron pulses preferably overlap with at least 70%, more preferably at least 80%, even more preferably at least 90% and most preferably 100% of the n bursts of the saturating electromagnetic field, B1-sat. The N hadron pulses are preferably in phase with the n bursts of the saturating electromagnetic field, B1-sat.

The N hadron pulses can have a period, PBi, comprised between 10 µs and 30 ms. Depending on the yype of hadron source, the period PBi can preferably be comprised between 1 and 10 ms or, alternatively, preferably between 5 and 20 ms. The time interval, ΔPBi, between two consecutive hadron pulses is preferably comprised between 1 and 20 ms. A short interval between hadron pulses is preferred to reduce the treatment time.

The period of each of the n bursts of the saturating electromagnetic field, B1-sat, is comprised preferably between 1 and 20 ms, preferably between 2 and 10 ms. The time period, Δts-e, separating the last burst of the n saturating electromagnetic field, B1-sat, and the first burst of the p exciting electromagnetic field, B1-exc, is either:
- not more than 50% of a longitudinal relaxation time, T1(A0), of the excitable atoms (A0) not affected substantially by the hadron beam, wherein Δts-e is preferably not more than 100 ms / T; or
- within ± 20% of the time, tM0, required for the longitudinal component, Mz, parallel to B0 of the net polarization vector of the irradiated excitable atoms to pass from the saturated state to a value of zero, and wherein Δts-e is preferably not more than 50 ms / T.

The **n** bursts of saturating electromagnetic field, B1-sat are preferably created as adiabatic bursts.

The target tissue can be a tumour exposed to a uniform magnetic field, B0, and traversed by a hadron beam of initial energy, E0. The frequency shift, ΔfLm, at the level of the Bragg peak of said hadron beam can be comprised between 60 and 6000 Hz, preferably between 200 and 1200 Hz in a main magnetic field, B0 = 1.5 T. The relative frequency shift, ΔfLmr = ΔfLm / flm0, can thus range from 0.9 to 93 ppm, preferably from 3 to 16 ppm.

The imaging volume, **Vp,** can be controlled by creating a magnetic gradient along, one, two, or three of the first, second, and third directions, **X1, X2, X3.** This way a thickness of the imaging volume along said first, second, or third directions, **X1, X2, X3** can be controlled.

A treatment session can be planned in two steps: first at time, t0, leading to the establishment of a treatment plan, and second at a time, t1 > t0, when a therapy session is to take place, and during which it is assessed whether the validity of the results established in the treatment plan are still applicable at time, t1. In particular, the method comprises the following steps:
(a) establishing at day, t0, a treatment plan and determining an initial energy, E0, of a hadron beam for depositing a given dose of hadrons to a target spot,
(b) comparing on the display the morphology and thicknesses of the tissues traversed by a hadron beam of initial energy, E0, at day, t1 > t0, with the morphology and thicknesses of the same tissues as defined in the treatment plan, at day, t0,
(c) visualizing on the same display the actual position of the Bragg peak of the hadron beam,
(d) in case of mismatch between the actual position of the Bragg peak and of the target tissue, correcting the initial energy, E1, of the hadron beam required for the Bragg peak to fall over the target spot.

The present invention also concerns a medical apparatus comprising the following components:
(a) a hadron source adapted for irradiating a target tissue (40) with a hadron beam (1h) having a beam energy, Ek, with k = 0 or 1, along a beam path in a number, N, of hadron pulses;
(b) a magnetic resonance imaging device (MRI) for the acquisition of magnetic resonance (MR) images within an imaging volume, Vp, including the target tissue (40), as the target tissue is being irradiated,
(c) a controller configured for controlling the hadron source and for acquiring magnetic resonance images by implementing the following steps:
   - creating a main magnetic field, B0, in the imaging volume, **Vp,** including the target tissue,
   - creating n bursts of a saturating electromagnetic field, **B1-sat,** wherein n is an integer greater than 0, which oscillates at a frequency range, **[fL1],** of band width, **b1** < **2·ΔfL40,** and centred on the Larmor irradiated frequency, **fL40,1,** and excluding the Larmor rest frequency, **fL40,0,** wherein
      o **fL40,0** is the rest Larmor frequency of excitable atoms present in the target tissue;
      o **fL40,1** is the irradiated Larmor frequency of irradiated excitable atoms (A1) defined as the excitable atoms present in the same target tissue, and exposed to the effects of a hadron beam of initial energy, E0, traversing said target tissue positioned in the same magnetic field, B0, and wherein
      o **ΔfL40** = **|fL40,1 - fL40,01;**
   - after a time, Δts-e, following the nth burst **B1-sat,** creating m bursts of an exciting electromagnetic field, **B1-exc,** oscillating at a frequency range, **[fL0],** centred on the Larmor rest frequency, **fL40,0,** wherein m is an integer greater than 0;
   - directing a hadron beam having the initial energy, **E0,** along a beam path intersecting said target tissue in a number, **N,** of hadron pulses of pulse periods, **Pbi ,** wherein, **N** is an integer greater than 0;
(d) a display for representing the target tissue from the magnetic resonance data acquired by the MRI within the imaging volume, **Vp,** and for visualizing the beam path in the target tissue as a hyposignal weaker than the signal generated by the excitable atoms which are not exposed significantly to the effects of the hadron beam,
   **characterized in that,** said controller is further configured for synchronizing the **N** hadron pulses to overlap with at least 50% of the **n** bursts of the saturating electromagnetic field, **B1-sat.**

### Brief description of the drawings

These and further aspects of the invention will be explained in greater detail by way of example and with reference to the accompanying drawings in which:
**Figure 1** shows a flowchart of a state of the art hadron therapy method using a hadron therapy device coupled to a MRI;
**Figure 2** schematically shows (a) the position of the Bragg peak of a hadron beam traversing tissues, (b) changes with time of the morphology and position of a target tissue can create a discrepancy between a treatment plan and an actual required treatment, and (c) relationship between actual path lengths and water equvialent path lengths.
**Figure 3** schematically shows two embodiments of a medical apparatus comprising a hadron therapy device coupled to a MRI;
**Figure 4** schematically illustrates the hadron pencil beam treatment of a target tissue;
**Figure 5** schematically shows a selection of an imaging slice, Vpi, in a MRI and creation of phase gradients and frequency gradients;
**Figure 6** shows two examples of an apparatus according to the present invention, showing access of a hadron beam to a target tissue;
**Figure 7** shows (a) magnetic data acquisition steps for imaging a volum by MRI, and (b) relaxation process of the spin of one excitable atom **A0.**
**Figure 8** shows the relaxation of an excited atom (a) from a saturated state at 180° with the spins of the atoms out of phase, and (b) from an excited state at 90° with the spin of the atoms in phase. **M** / **M0** is the relative magnetic moment, with **M0** beng the maximum value of said magnetic moment, **M.**
**Figure 9** shows (a) the frequency shift, **ΔfLm,** between the irradiated and rest Larmor frequencies, **fLm1** and **fLm0,** of an excitable atom irradiated or not by a hadron beam, (b) the sequences of the excitation step, MRe, for acquiring MR data, (c) the pulses of hadron beam required for visualizing at least part of the beam path of the hadron beam, and (d) the spins of the excitable atoms A0 and A1 at the different stages of the excitation step.

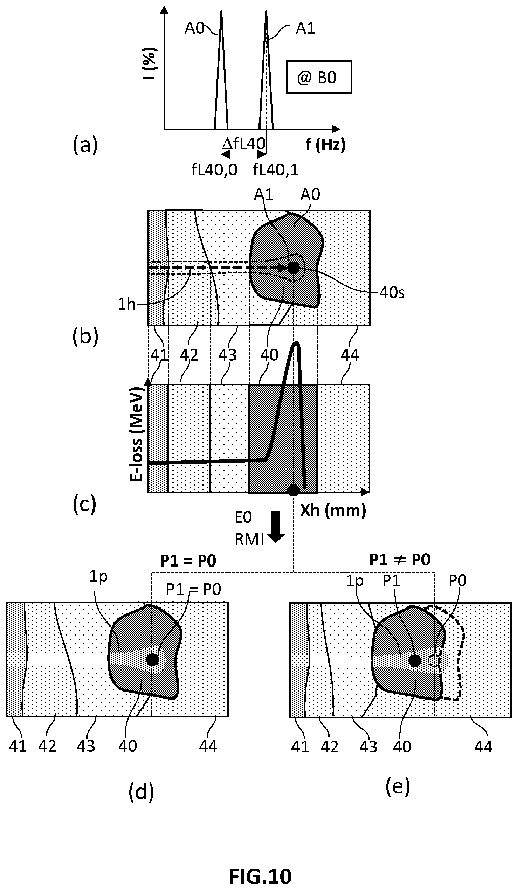

**Figure 10** shows (a) the frequency shift, **ΔfL40,** between the irradiated and rest Larmor frequencies, **fL40,1** and **fL40,0,** of an excitable atom in a target tissue irradiated or not by a hadron beam, (b) a cut of a tissues traversed by a hadron beam from an upstream boundary to a target spot, with the localization of the irradiated excitable atoms A1 indicated with dashed lines, (c) the corresponding **Eₗₒₛₛ** curve of the hadron beam represented in (b), and (d) a schematic representation of a RMI image obtained by the present invention with the beam path of the hadron beam visible as a hyposignal.
**Figure 11** shows a flowchart of a hadron therapy method according to the present invention.

The figures are not drawn to scale.

### Detailed description of preferred embodiments

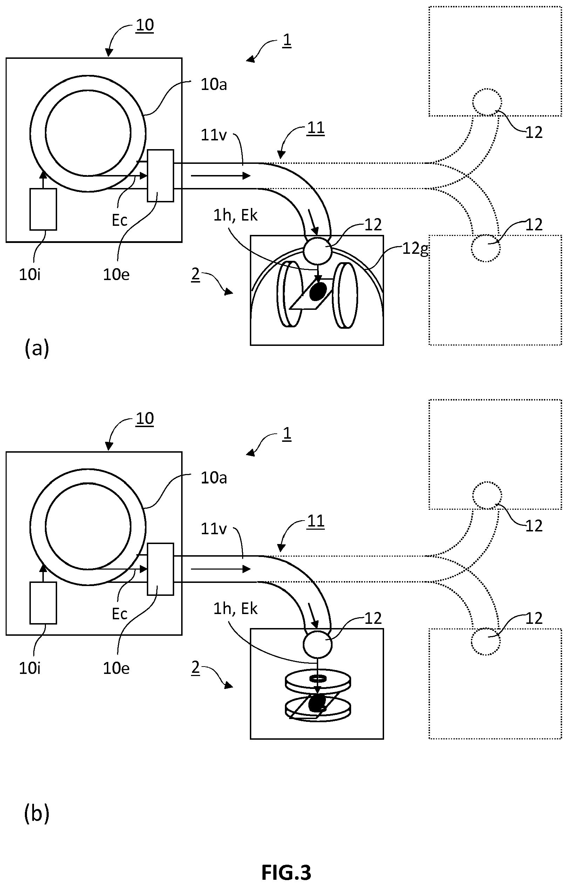

Figure 3 illustrates two examples of a medical apparatus comprising a hadron therapy device **1** coupled to a magnetic resonance imaging device (MRI) **2** according to the present invention. A hadron therapy device and a MRI and the combination of the two are described more in details in the following.

### Hadron therapy device

Hadron therapy is a form of external beam radiotherapy using beams **1h** of energetic hadrons. Figures 3, 4&6 show a hadron beam **1h** directed towards a target spot **40s** in a target tissue **40** of a subject of interest. Target tissues **40** of a subject of interest typically include cancerous cells forming a tumour. During a hadron therapy session, a hadron beam of initial energy, **Ek,** with k = 0 or 1, irradiates one or more target spots within the target tissue, such as a tumour, and destroys the cancerous cells included in the irradiated target spots, thus reducing the size of the treated tumour by necrosis of the irradiated tissues.

The subject of interest may comprise a plurality of materials including organic materials. Preferably, the subject of interest comprises a plurality of tissues **m,** with m = 40-44 as shown in Figure 2, that can be, for example: skin, fat, muscle, bone, air, water (blood), organ, and tumour. The target tissue 40 is preferably a tumour.

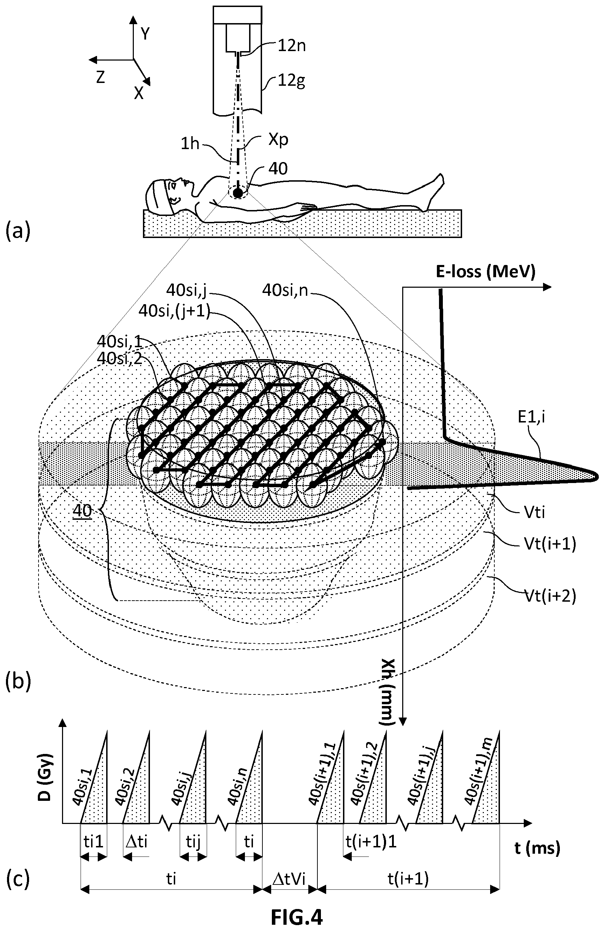

As described in the prior art literature, a hadron beam **1h** traversing an organic body along a beam path, **Xp,** loses most of its energy at a specific distance of penetration along the beam path, **Xp.** As illustrated in Figures 2&4, said specific distance of penetration corresponds to the position of the so called Bragg peak, observed when plotting the energy loss per unit distance [MeVg⁻¹cm⁻²], **Eₗₒₛₛ,** of a hadron beam as a function of the distance, **xh,** measured along the beam path, **Xp.** Unlike other forms of radiation therapies, a hadron beam can thus deliver a high dose of energy at a very specific location within a target tissue corresponding to the position of the Bragg peak. The position of the Bragg peak depends mainly on the initial energy, **Ek,** of the hadron beam (i.e., before traversing any tissue) and on the nature and thicknesses of the traversed tissues. The hadron dose delivered to a target spot depends on the intensity of the hadron beam and on the time of exposure. The hadron dose is measured in Grays (Gy), and the dose delivered during a treatment session is usually of the order of one to several Grays (Gy).

A hadron is a composite particle made of quarks held together by strong nuclear forces. Typical examples of hadrons include protons, neutrons, pions, and heavy ions, such as carbon ions. In hadron therapy, electrically charged hadrons are generally used. Preferably, the hadron is a proton and the corresponding hadron therapy is referred to as proton therapy. In the following, unless otherwise indicated, any reference to a proton beam or proton therapy applies to a hadron beam or hadron therapy in general.

A hadron therapy device **1** generally comprises a hadron source **10,** a beam transport line **11,** and a beam delivery system **12.** Charged hadrons may be generated from an injection system **10i,** and may be accelerated in a particle accelerator **10a** to build up energy. Suitable accelerators include for example, a cyclotron, a synchro-cyclotron, a synchrotron, or a laser accelerator. For example, a (synchro-)cyclotron can accelerate charged hadron particles from a central area of the (synchro-)cyclotron along an outward spiral path until they reach the desired output energy, **Ec,** whence they are extracted from the (synchro-)cyclotron. Said output energy, **Ec,** reached by a hadron beam when extracted from the (synchro-)cyclotron is typically comprised between 60 and 400 MeV, preferably between 210 and 250 MeV. The output energy, **Ec,** is not necessarily the initial energy, **Ek,** of the hadron beam used during a therapy session; **Ek** is equal to or lower than **Ec, Ek ≤ Ec.** An example of a suitable hadron therapy device includes, but is not limited to, a device described in US Pat. No.4870287, the entire disclosure of which is incorporated herein by reference as representative of a hadron beam therapy device as used in the present invention.

The energy of a hadron beam extracted from a (synchro-)cyclotron can be decreased by energy selection means **10e** such as energy degraders, positioned along the beam path, **Xp,** downstream of the (synchro-)cyclotron, which can decrease the output energy, **Ec,** down to any value **of Ek** including down to nearly 0 MeV. As discussed supra, the position of the Bragg peak along a hadron beam path, **Xp,** traversing specific tissues depends on the initial energy, **Ek,** of the hadron beam. By selecting the initial energy, **Ek,** of a hadron beam intersecting a target spot **40s** located within a target tissue, the position of the Bragg peak can be controlled to correspond to the position of the target spot.

A hadron beam can also be used for characterizing properties of tissues. For example, images can be obtained with a hadron radiography system (HRS or, in particular a proton radiography system, PRS). The doses of hadrons delivered to a target spot for characterization purposes, however, may be considerably lower than the doses delivered during a hadron therapy session which, as discussed supra, are of the order of 1 to 10 Gy. The doses of delivered hadrons of HRS for characterization purposes are typically of the order of 10⁻³ to 10⁻¹ Gy (i.e. one to four orders of magnitude lower than doses typically delivered for therapeutic treatments). These doses have no significant therapeutic effects on a target spot. Alternatively, a treatment hadron beam delivered to a small set of target spots in a target tissue may be used for characterization purposes. The total dose delivered for characterization purposes is not sufficient to treat a target tissue.

As illustrated in Figure 3, downstream of the hadron source, a hadron beam of initial energy, **Ek,** is directed to the beam delivery system **12** through a beam transport line **11.** The beam transport line may comprise one or more vacuum ducts, **11v,** a plurality of magnets for controlling the direction of the hadron beam and/or for focusing the hadron beam. The beam transport line may also be adapted for distributing and/or selectively directing the hadron beam from a single hadron source **10** to a plurality of beam delivery systems for treating several patients in parallel.

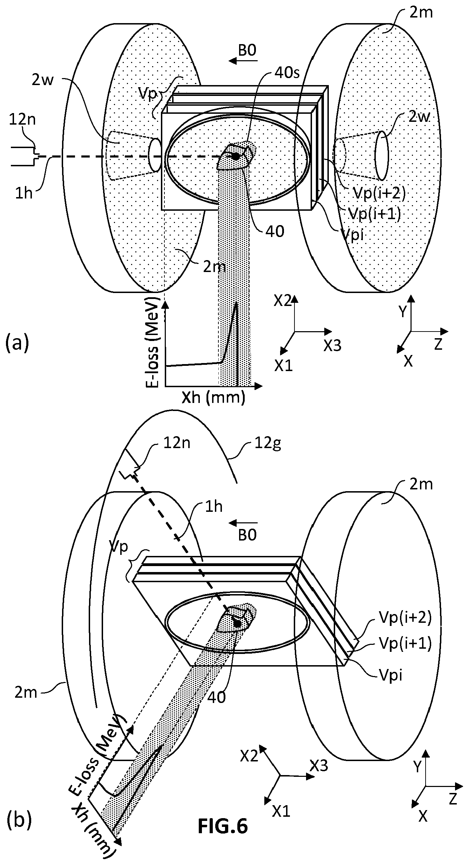

The beam delivery system **12** comprises a nozzle **12n** for orienting a hadron beam **1h** along a beam path, **Xp.** The nozzle can either be fixed or mobile. Mobile nozzles are generally mounted on a gantry **12g** as illustrated schematically in Figures 4&6. A gantry is used for varying the orientation of the hadron outlet about a circle centred on an isocentre and normal to an axis, **Z,** which is generally horizontal. In supine hadron treatment devices, the horizontal axis, **Z,** may be selected parallel to a patient lying on a couch (i.e. the head and feet of the patient are aligned along the horizontal axis, **Z**)**.** The nozzle **12n** and the isocentre define a path axis, **Xn,** which angular orientation depends on the angular position of the nozzle in the gantry. By means of magnets positioned adjacent to the nozzle, the beam path, **Xp,** of a hadron beam **1h** can be deviated with respect to the path axis, **Xn,** within a cone centred on the path axis and having the nozzle as apex (cf. Figure 4(a)). This is advantageous in that a volume of target tissue centred on the isocentre can be treated by hadron beam without changing the position of the nozzle within the gantry. The same applies to fixed nozzles with the difference that the angular position of the path axis is fixed.

A target tissue to be treated by a hadron beam in a device provided with a gantry must be positioned near the isocentre. To this purpose, the couch or any other support for the patient can be moved; it can typically be translated over a horizontal plane **(X, Z)** wherein **X** is a horizontal axis normal to the horizontal axis, **Z,** and translated over a vertical axis, **Y,** normal to **X** and **Z,** and can also be rotated about any of the axes **X, Y, Z,** so that a central area of the target tissue can be positioned at the isocentre.

To assist in the correct positioning of a patient with respect to the nozzle **12n** according to a treatment plan previously established, the beam delivery system may comprise imaging means. For example, a conventional X-ray radiography system can be used to image an imaging volume, **Vp,** comprising the target tissue **40.** The thus obtained images can be compared with corresponding images collected previously during the establishment of the treatment plan.

Depending on the pre-established treatment plan, a hadron treatment may comprise delivery of a hadron beam to a target tissue in various forms, including the following techniques well known in the art: pencil beam, single scattering, double scattering, and uniform scattering. The present invention may apply to all hadron therapy technique. The hadron treatment, however, is preferably applied by a pencil beam technique. Figure 4 illustrates schematically this technique of delivery. A hadron beam of initial energy, **Ek**,**1**, is directed to a first target spot **40s1**,**1**, during a pre-established delivery time. The hadron beam is then moved to a second target spot **40s1,2,** during a pre-established delivery time. The process is repeated on a sequence of target spots **40s1,j** to scan a first iso-energy treatment volume, **Vt1**, following a pre-established scanning path. A second iso-energy treatment volume, **Vt2,** is scanned spot by spot following a similar scanning path with a hadron beam of initial energy, **Ek,2.** As many iso-energy treatment volumes, **Vti,** as necessary to treat a given target tissue **40** are thus irradiated following a similar scanning path. A scanning path can include several passages over a same scanning spot **40si**,**j**. The iso-energy treatment volumes, **Vti,** are volumes of target tissues which can be treated with a hadron beam of initial energy, **Ek,i.** The iso-energy treatment volumes, **Vti,** are slice shaped, with a thickness corresponding approximately to the breadths of the Bragg peaks at the values of the initial energy, **Ek,i,** of the corresponding hadron beams, and with main surfaces of area only limited by the opening angle of the cone centred on the path axis, **Xn,** enclosing the beam paths, **Xp,** available for a given position of the nozzle in the gantry or in a fixed nozzle device. In case of a homogeneous target tissue, the main surfaces are substantially planar as illustrated in Figure 4(b). In reality, however, since both target tissue **40** and upstream tissues **41-43** are not homogeneous in nature and thickness, the main surfaces of an iso-energy volume, **Vti,** are bumpy. The egg-shaped volumes in Figure 4(b) schematically illustrate the volumes of target tissue receiving a therapeutic dose of hadron by exposure of one target spot **40si,j** to a beam of initial energy **Ek,i.**

The dose, **D,** delivered to a target tissue **40** is illustrated in Figure 4(c). As discussed supra, the dose delivered during a treatment session is usually of the order of one to several Grays (Gy). It depends on the doses delivered to each target spot **40si,j,** of each iso-energy treatment volume, **Vti.** The dose delivered to each target spot **40si,j** depends on the intensity, **I,** of the hadron beam and on the irradiation time **tij** on said target spot. The dose, **Dij,** delivered to a target spot **40si,j** is therefore the integral, **Dij** = ∫**I** dt, over the irradiation time **tij.** A typical dose, **Dij,** delivered to a target spot **40si,j** is of the order of 0.1-20 cGy. The dose, **Di,** delivered to an iso-energy treatment volume, **Vti,** is the sum over the **n** target spots scanned in said iso-energy treatment volume of the doses, **Dij,** delivered to each target spot, **Di** = ∑ **Dij,** for **j** = 1 to n. The total dose, **D,** delivered to a target tissue **40** is thus the sum over the **p** irradiated iso-energy treatment volumes, **Vti,** of the doses, **Di,** delivered to each energy treatment volume, **D** = ∑ Di, for i = 1 to q. The dose, **D,** of hadrons delivered to a target tissue can therefore be controlled over a broad range of values by controlling one or more of the intensity, **I,** of the hadron beam, the total irradiation time **tij** of each target spot **40si,j,** and the number of irradiated target spots **40si**,**j**. Once a patient is positioned such that the target tissue **40** to be treated is located at the approximate position of the isocentre the duration of a hadron treatment session therefore depends mainly on the values of:
- the irradiation time, **tij,** of each target spot **40si,j,**
- the scanning time, **Δti,** for directing the hadron beam from a target spot **40si,j** to an adjacent target spot **40si(j+1)** of a same iso-energy treatment volume, **Vti,**
- the number n of target spots **40si,j** scanned in each iso-energy treatment volume, **Vti,**
- the time, **ΔtVi,** required for passing from a last target spot **40si,n** scanned in an iso-energy treatment volume, **Vti,** to a first target spot **40s(i+1),1** of the next iso-energy treatment volume, **Vt(i+1),** and
- the number of iso-energy treatment volumes, **Vti,** in which a target tissue **40** is enclosed.

The irradiation time, **tij,** of a target spot **40si,j** is of the order of 1-20 ms. The scanning time, **Δti,** between successive target spots in a same iso-energy treatment volume is generally very short, of the order of 1 ms (up to about 20 ms). The time, **ΔtVi,** required for passing from one iso-energy treatment volume, **Vti,** to a subsequent iso-energy treatment volume, **Vt(i+1),** is slightly longer because it requires changing the initial energy, **Ek,** of the hadron beam and is of the order of 1-2 s.

As illustrated in Figure 2(a)&(b), an accurate determination of the initial energy, **Ek,** of a hadron beam is clearly critical, because if the position of the Bragg peak thus determined does not correspond to the actual position of the target tissue **40,** substantial doses of hadrons could be delivered to healthy, sometimes vital organs and could possibly endanger the health of a patient. The position of the Bragg peak mainly depends on the initial energy, **Ek,** of the hadron beam and on the nature and thicknesses of the traversed tissues. Besides determining the exact position of the target tissue within a patient, the computation of the initial energy, **Ek,** of a hadron beam yielding a position of the Bragg peak corresponding to the precise position of the target tissue therefore also requires the preliminary characterization of the tissues traversed until reaching the target tissue **40.** This characterization is performed during a treatment plan established before (generally several days before) the actual hadron treatment. The actual hadron treatment can be divided in several sessions distributed over several weeks. A typical treatment plan may start by the acquisition of data, generally in the form of images of the subject of interest with a CT scan. The images thus acquired by a CT scan may be characterized, by performing one or more of the steps:
- identifying the nature of the tissues represented on the images as a function of the X-rays absorption power of the tissues, based on the comparison of shades of grey of each tissue with a known grey scale; for example, a tissue can be one of fat, bone, muscle, water, air;
- measuring the positions and thicknesses of each tissue along one or more hadron beam paths, **Xp,** from the skin to the target tissue;
- based on their respective nature, attributing to each identified tissue a corresponding hadron stopping power ratio (HSPR);
- calculating a tissue water equivalent path length, WEPLm, of each tissue **m,** with **m** = 40 to 44, upstream of and including the target tissue, based on their respective HSPR and thicknesses;
- adding the thus determined WEPLm of all tissues **m** to yield a WEPL40s, of a target spot **40s** located in the target tissue **40,** said WEPL40s corresponding to the distance travelled by hadron beam from the skin to the target spot **40s;**
- from the WEPL40s, calculating the initial energy **Ek** of a hadron beam required for positioning the Bragg peak of the hadron beam at the target spot **40s.**

Said process steps can be repeated for several target spots defining the target tissue.

### Magnetic resonance imaging device

A magnetic resonance imaging device **2** (MRI) implements a medical imaging technique based on the interactions of excitable atoms present in an organic tissue of a subject of interest with electromagnetic fields. When placed in a strong main magnetic field, **B0,** the spins of the nuclei of said excitable atoms precess around an axis aligned with the main magnetic field, **B0,** resulting in a net polarization at rest that is parallel to the main magnetic field, **B0**. The application of a pulse of radio frequency (RF) exciting magnetic field, **B1,** at the frequency of resonance, **fL,** called the Larmor frequency, of the excitable atoms in said main magnetic field, **B0,** excites said atoms by tipping the net polarization vector sideways (e.g., with a so-called 90° pulse, **B1-90**) or to angles greater than 90° and even reverse it at 180° (with a so-called 180° pulse, **B1-180**). When the RF electromagnetic pulse is turned off, the spins of the nuclei of the excitable atoms return progressively to an equilibrium state yielding the net polarization at rest. During relaxation, the transverse vector component of the spins produces an oscillating magnetic field inducing a signal which can be collected by antennas **2a** located in close proximity to the anatomy under examination.

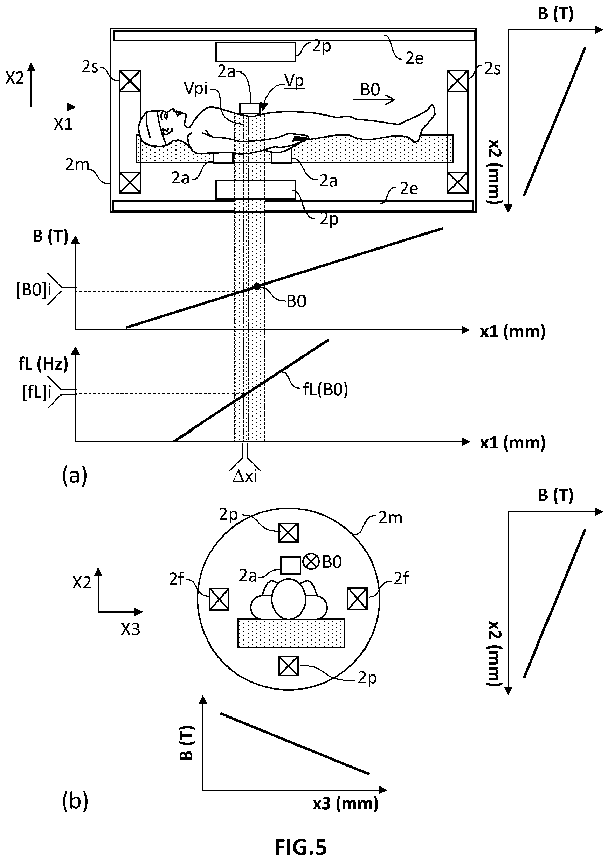

As shown in Figures 5 and 6, a MRI **2** usually comprises a main magnet unit **2m** for creating a uniform main magnetic field, **B0;** radiofrequency (RF) excitation coils **2e** for creating the RF-exciting magnetic field, **B1;** X1-, X2-, and X3-gradient coils, **2s, 2p, 2f,** for creating magnetic gradients along the first, second, and third directions **X1, X2,** and **X3,** respectively; and antennas **2a,** for receiving RF-signals emitted by excited atoms as they relax from their excited state back to their rest state. The main magnet produces the main magnetic field, **B0,** and can be a permanent magnet or an electro-magnet (a supra-conductive magnet or not). An example of a suitable MRI includes, but is not limited to, a device described in US Pat. No. 4694836, the entire disclosure of which is incorporated herein by reference.

As illustrated in Figure 5, an imaging slice or layer, **Vpi,** of thickness, **Δxi**, normal to the first direction, **X1,** can be selected by creating a magnetic field gradient along the first direction, **X1.** In Figure 5, the first direction, **X1,** is parallel to the axis Z defined by the lying position of the patient, yielding slices normal to said axis Z. In practice, this is not necessarily the case, and the first direction, **X1,** can be any direction, e.g. transverse to the axis Z, with slices extending at an angle with respect to the patient. As shown in Figure 5(a), because the Larmor frequency, **fL,** of an excitable atom depends on the magnitude of the magnetic field it is exposed to, sending pulses of RF exciting magnetic field, **B1,** at a frequency range, **[fL]i,** excites exclusively the excitable atoms which are exposed to a magnetic field range, [**B0**]**i**, which are located in a slice or layer, **Vpi,** of thickness, **Δxi.** By varying the frequency bandwidth, **[fL]i,** of the pulses of RF exciting magnetic field, **B1,** the width, **Δxi**, and position of an imaging layer, **Vpi,** can be controlled. By repeating this operation on successive imaging layers, **Vpi,** an imaging volume, **Vp,** can be characterized and imaged.

To localize the spatial origin of the signals received by the antennas on a plane normal to the first direction, **X1,** magnetic gradients are created successively along second and third directions, **X2, X3,** wherein **X1** ⊥ **X2** ⊥ **X3,** by activating the X2-, and X3-gradient coils **2p, 2f,** as illustrated in Figure 5(b). Said gradients provoke a phase gradient, **Δϕ**, and a frequency gradient, **Δf**, in the spins of the excited nuclei as they relax, which allows spatial encoding of the received signals in the second and third directions, **X2, X3.** A two-dimensional matrix is thus acquired, producing *k*-space data, and an MR image is created by performing a two-dimensional inverse Fourier transform. Other modes of acquiring and creating an MR image are known in the art and the present invention is not restricted to the selection of any particular mode.

The main magnetic field, **B0,** is generally comprised between 0.2 and 7 T, preferably between 1 and 4 T. The radiofrequency (RF) excitation coils **2e** generate a magnetic field at a frequency range, **[fL]i,** around the Larmor frequencies, **fL,** of the atoms comprised within a slice of thickness, **Δxi**, and exposed to a main magnetic field range **[B0i].** For atoms of hydrogen, the Larmor frequency per magnetic strength unit, **fL** / **B** = 42.6 MHz T⁻¹. For example, for hydrogen atoms exposed to a main magnetic field, **B0** = 2 T, the Larmor frequency, **fL** = 85.2 MHz.

The MRI can be any of a closed-bore, open-bore, or wide-bore MRI type. A typical closed-bore MRI has a magnetic strength of 1.0 T through 3.0 T with a bore diameter of the order of 60 cm. An open-bore MRI, as illustrated in Figure 6, has typically two main magnet poles **2m** separated by a gap between them for accommodating a patient in a lying position, sitting position, or any other position suitable for imaging an imaging volume, **Vp.** The magnetic field of an open-bore MRI is usually comprised between 0.2 and 1.0 T. A wide-bore MRI is a kind of closed-bore MRI having a larger diameter.

### Hadron therapy device + MRI

As discussed in the introduction with reference to Figure 2(b), the position and morphology of a target tissue **40** can evolve between a time, **t0**, of establishment of a treatment plan and a time, **t1** = **t0** + **Δt3,** of a treatment session, which can be separated by several days or weeks. A target spot **40si,j** identified in the treatment plan as belonging to the target tissue **40p** may not belong to the target tissue **40** anymore at the time, **t0** + **Δt3,** of the treatment session. The irradiation of said target spot may harm healthy tissues **43** instead of target tissues **40.**

To avoid such incidents, the prior art proposes coupling a hadron therapy device (PT) **1** to an imaging device, such as a magnetic resonance imaging device (MRI) **2.** Such coupling may not be trivial with a number of challenges to overcome but PT-MRI apparatuses have been described in the recent art and are generally known by the persons of ordinary skill in the art. For example, solutions to problems such as the correction of a hadron beam path, Xp, within a strong magnetic field, **B0,** of the MRI are available.

A PT-MRI apparatus allows the morphologies and positions of the target tissue and surrounding tissues to be visualized the day, **t0** + **Δt3,** of the treatment session for comparison with the corresponding morphologies and positions acquired during the establishment of a treatment plan at time, **t0.** As illustrated in the flowchart of Figure 1, in case a discrepancy of the tissues morphologies and positions between the establishment of the treatment plan at time, **t0**, and the treatment session at time, **t0** + **Δt3,** was observed, the treatment session would be interrupted and a new treatment plan would probably be established with the definition of new target spots corresponding to the actual target tissue **40** to be irradiated by hadron beams of corrected energies and directions (cf. Figure 1, diamond box "∃Δ?" → Y → "STOP"). This development of the prior art represents already a major improvement over carrying out a hadron therapy session based solely on information collected during the establishment of the treatment plan at time, **t0**, which may be obsolete at the time, **t0** + **Δt3,** of the treatment session.

The present invention aims at further improving the efficacy of a PT-MRI apparatus by providing the information required for correcting *in situ* the initial energies, **Ek,** and beam path, **Xp,** directions of the hadron beams, in case a change of morphology or position of the target tissue were detected. This would allow the treatment session to take place in spite of any changes detected in the target tissue **40.**

The MRI used can be any of a closed-bore, open-bore, or wide-bore MRI type described above. An open MRI affords much open space in the gap separating the two main magnet poles **2m** for orienting a hadron beam in almost any direction. Alternatively, openings or windows **2w** transparent to hadrons can be provided on the main magnet units as illustrated in Figure 6(a). This configuration has the particularity that the hadron beam can be parallel to B0. In another embodiment, a hadron beam can be oriented through the cavity of the tunnel formed by a closed bore MRI, or an annular window transparent to hadrons may extend parallel to a gantry substantially normal to the axis Z, over a wall of said tunnel, such that hadron beams may reach a target tissue with different angles. In case a fixed nozzle is used, the size of such opening or window can be reduced accordingly.

### MRI imaging of tissues and of hadron beam

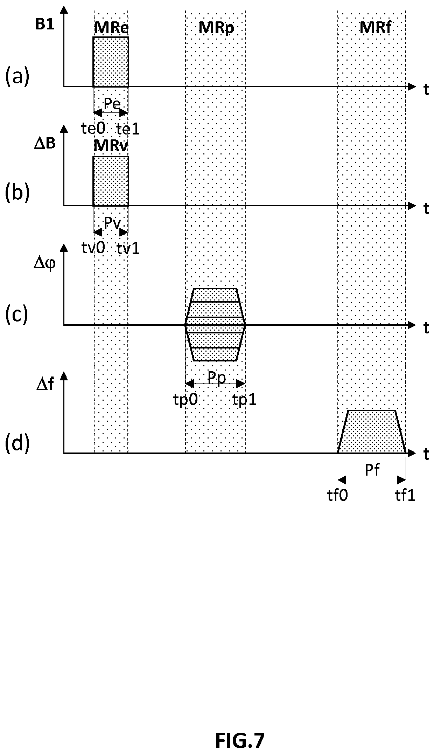

In a nutshell, acquisition of magnetic resonance data by a MRI for imaging a volume, **Vp,** comprises the following steps illustrated in Figure 7(a).
- an **excitation step** (MRe) for exciting the spin of the nuclei of excitable atoms **A0,** generally hydrogen; as shown in Figure 7(a), the excitation step (MRe) is applied during a period, **Pe** = **te1 - te0;**
- a **layer selection step** (MRv) for selecting an imaging layer, **Vpi,** of the imaging volume, **Vp,** of thickness, **Δxi**, measured along the first direction, **X1;** as shown in Figure 7(b), the layer selection step (MRv) is applied during a period, **Pv** = **tv1 - tv0,** wherein the periods **Pe** and **Pv** are substantially simultaneous and equal;
- a **phase gradient step** (MRp) for localising along the second direction, **X2,** the origin of RF signals received by the antennas during relaxation of the excited spins; as shown in Figure 7(c), the phase gradient step (MRp) is applied during a period, **Pp** = **tp1 - tp0,** wherein generally, **tp0** ≥ **te1;** and
- a **frequency gradient step** (MRf)) for localising along the third direction, **X3,** the origin of RF signals received by the antennas during relaxation of the excited spins; as shown in Figure 7(d), the frequency gradient step (MRf) is applied during a period, **Pf = tf1 - tf0,** wherein generally, **tf0** ≥ **tp1**.

The excitation step comprises creating pulses of an excitation electromagnetic field, **B1,** with the RF unit **2e** oscillating at a RF-frequency range, **[fL]i,** during an excitation period, **Pe.** The excitable atoms **A0** present in an imaging layer, **Vpi,** of thickness, **Δxi**, are excited at their Larmor frequency which depends on the strength of the magnetic field they are exposed to which corresponds to a magnetic field range, **[B0]i** = [**Bi**,**0**, **Bi,1]** controlled by the magnetic gradient created along the first direction, X1 (and/or other directions) (cf. Figure 5(a)). The thickness, **Δxi**, can be varied as a function of the slope of the magnetic gradient and, in particular, by varying the band width of the RF-frequency range, **[fL]i,** applied by the RF unit **2e.** Depending on the sequence and intensity of the RF-excitation electromagnetic field, **B1,** different types of excitations can be imposed onto the excitable atoms. The imaging volume, **Vp,** is genreally divided into several imaging layers, **Vpi,** which sizes can be restricted along three dimensions by creating a magnetic gradient along, one, two, or three of the first, second, and third directions, X1, X2, X3. The thickness of the imaging volume can thus be controlled along said first, second, or third directions, X1, X2, X3, to define a slice (restricted over one direction only), an elongated prism (restricted over two directions), or a box (restricted over the three directions X1, X2, X3.

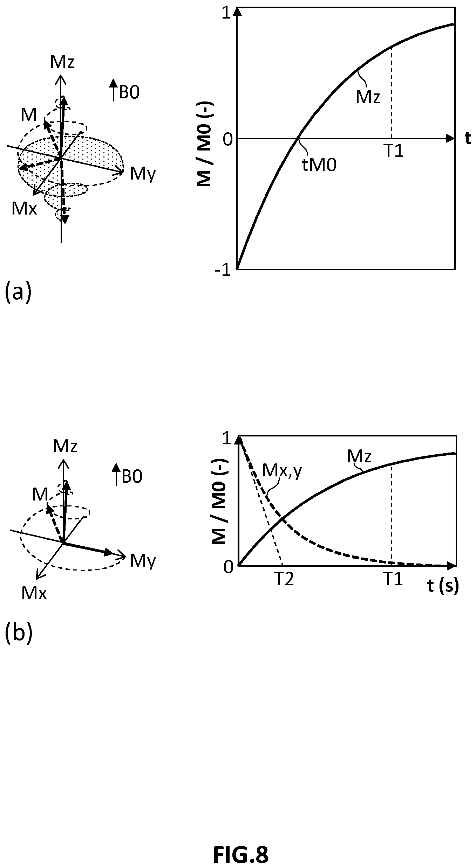

As illustrated in Figure 8, absent an excitation electromagnetic field, **B1,** the net polarization vector of the excitable atoms **A0** (generally hydrogen) of a tissue exposed to a main magnetic field, **B0,** parallel to the axis **Z,** is parallel to both **B0** and **Z,** with a net polarization component, Mx,y, in the directions X and Y which is zero as the spins precessing about the axis **Z** are out of phase and compensate each other. Upon excitation at their Larmor frequencies with an excitation electromagnetic field, **B1,** the precessing angle of the spins increases yielding a decrease in the Z-component, **Mz,** of the net polarization vector. Depending on the type of RF-excitation the spins can be brought in phase or not. In the former case, the net polarization component, Mx,y, increases as the spins of the excited atoms precess in phase. Figure 8(b) illustrates an excitable atom excited at 90°, yielding a zero Mz-component and maximum Mx,y-components, and relaxing back to its rest state after the end of the excitation. The relaxation process and corresponding relaxation times, T1, T2 are illustrated in the adjacent graph. Figure 8(a) illustrates an excitable atom excited at 180°. As the spin cannot be more excited than at 180°, this excited state is commonly referred to as a saturated state. In practice, and as applied in the present invention, a saturated state is defined as an excitation state at an angle of at least 100° (and up to 180°). The corresponding relaxation process and relaxation times, T1, T2 are illustrated in the adjacent graph of Figure 8(a).

Although much desired, visualization by MRI of a hadron beam traversing tissues comprised within a MRI imaging volume, **Vp,** is not straightforward and has, to our knowledge, not yet been reported. Even proton beams, corresponding to hydrogen nuclei, cannot be visualized by MRI under normal conditions. The present invention defines specific conditions allowing a hadron beam and, in particular, the position of the Bragg peak of said hadron beam to be identifiable on a MRI image of an imaging volume, **Vp,** traversed by said hadron beam.

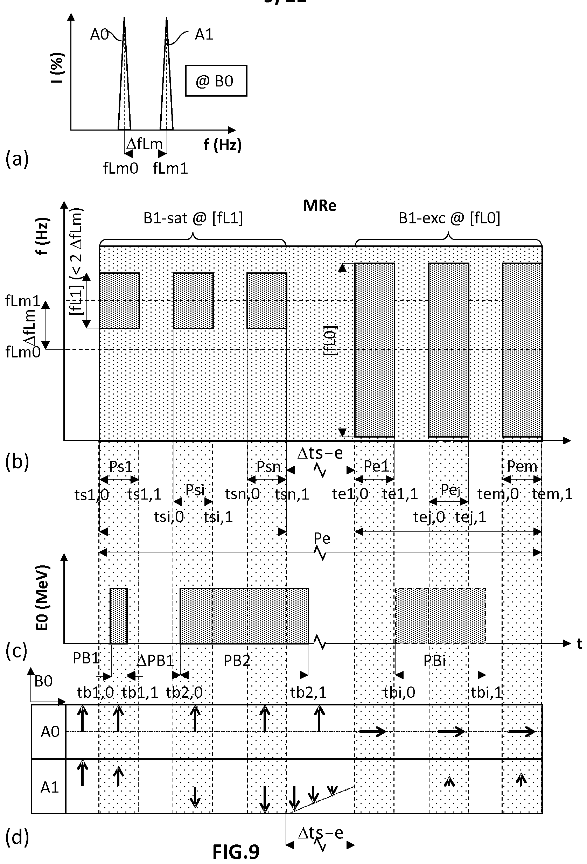

The solution proposed herein is based on the observation illustrated in Figure 9(a), that the Larmor rest frequency, **fLm0** of an excitable atom **A0,** such as hydrogen, in a tissue **m** exposed to a main magnetic field, **B0,** shifted to a value, **fLm1,** of a Larmor irradiated frequency, when said excitable atom interacted with a hadron beam passing in its direct vicinity (such atom is herein referred to as an irradiated excitable atom **A1**). The magnitude of the shift of the Larmor frequency can be expressed as an absolute value, **ΔfLm** = **|fLm1 - fLm0|** expressed in Hz, or as a relative value **ΔfLmr** = **ΔfLm** / **fLm0,** expressed in ppm. Whilst the relative value, **ΔfLmr,** is substantially independent of the magnetic field, **B0,** the magnitude of the shift, **ΔfLm,** depends on the strength of the main magnetic field, **B0,** and, in a minor scale, on the nature of the tissue **m** comprising the excitable atoms and being traversed by a hadron beam. For example the value of the shift, **ΔfLm,** of the Larmor frequency can be comprised between 60 and 6000 Hz, preferably between 200 and 1200 Hz in a main magnetic field, **B0** = 1.5 T, corresponding to a relative shift, **ΔfLmr,** of about 0.9 to 93 ppm, preferably of about 3 to 16 ppm.

Without wishing to be bound by any theory, the magnetic susceptibility of excitable atoms **A0** is modified by the effect of a hadron beam, yielding irradiated excitable atoms **A1**. The concentration of irradiated excitable atoms A1 is a function of the energy deposited by said hadron beam in the tissues it traverses, As illustrated in Figure 2, a hadron beam deposits almost all its energy at the level of the Bragg peak, which is quite narrow. The magnetic susceptibility of the excitable atoms on and adjacent to the hadron beam path therefore varies most at the level of the Bragg peak, resulting in a higher concentration of irradiated excitable atoms **A1** at said level of the Bragg peak. Based on the treatment plan established earlier, the Bragg peak must be located within a target tissue **40** surrounding a target spot **40s.** The present invention takes advantage of the foregoing mechanism for visualizing in a MR image of an imaging volume, **Vp,** of tissues at least the portion of the hadron beam at the level of the Bragg peak in the target tissue **40** and, possibly the whole of the hadron beam path from the outer surface of the skin of a patient to the target tissue.

The gist of the present invention lies, on the one hand, on the specific sequence applied in the excitation step, **MRe,** for the MR data acquisition as a function of the shift, **ΔfLm,** of the excitable atoms in a specific target tissue **40** and, on the other hand, on a specific synchronization of the hadron beam with the excitation step. The shift, **ΔfLm,** can be measured by nuclear resonance spectroscopy (MNR) the peaks corresponding to the excitation of excitable atoms A0 of the target tissue at rest and of irradiated excitable atoms A1 exposed to a hadron beam, yielding a spectrum as schematically illustrated in Figure 9(a). Upon increasing the number of such RMN measurements, databases will soon be available giving good approximations of the values of, **ΔfLm,** as a function of the tissues and of the main magnetic field, **B0**.

The excitation step, **MRe,** comprises two main steps as illustrated in Figure 9(b). First, a A1-saturation step is performed on the irradiated excitable atom **A1**. The A1-saturation step comprises emitting n bursts of a saturating electromagnetic field, **B1-sat,** which oscillates at a frequency range, **[fL1],** of band width, **b1** < **2·ΔfL40,** and centred on the Larmor irradiated frequency, **fL40,1.** It is important that the band width **b1** excludes the Larmor rest frequency, **fL40,0.** The number **n** of bursts is an integer greater than 0. The boundary of the frequency range, **[fl1],** closest to the Larmor frequency **fLm0** of the excitable atoms **A0** is separated from the latter by a value of preferably at least ½ **ΔfLm.** The expression *"centred on the [...] frequency **jL40,1**"* is not meant to restrict the position of **fl40,1** to exactly the mid-point of the frequency range **[fL1],** but is meant to indicate that the Larmor frequency **fL40,1** is comprised within a mid-portion of the range **[fL1],** rather separate from the upper and lower boundaries of said range. For example the Larmor frequency **fL40,1** can be separated from the upper and lower boundaries of **[fL1]** by at least 20% of the range **[fL1].** The period, **Psi** = **tsi1 - tsi0,** of the bursts of **B1-sat** can be comprised between 1 and 20 ms, preferably between 5 and 15 ms. They can be repeated at intervals, **(ts(i+1)0 - tsi1),** comprised between 1 and 50 ms, preferably between 5 and 20 ms.

The saturating electromagnetic field, **B1-sat,** brings the nuclei of the irradiated excitable atoms (**A1**) to a saturated state wherein a net polarization vector of the spins is reversed at an angle comprised between 100 and 180° with respect to the net polarization vector of the spins of said nuclei at rest (i.e., absent **B1-sat**). As illustrated in Figure 8(a), a reversed angle of 180° is preferred for enhancing the visibility of the hadron beam path, but lower angles can be advantageous too, as they reduce the data acquisition times. In a preferred embodiment, the spins of the saturated atoms are not brought into phase by the **n** bursts of **B1-sat,** such that the X- and Y-components, **Mx,y,** of the net polarization vector in the X- and Y-directions remain substantially zero in the saturated state; this configuration is represented in the graph of Figure 8(a).

At the end of the A1-saturation step, an A0-excitation step is performed for exciting the excitable atoms **A0,** which are not substantially affected by the passage of the hadron beam. The A0-excitation step comprises creating **p** bursts of an exciting electromagnetic field, **B1-exc,** oscillating at a frequency range, **[fL0],** and centred on the Larmor rest frequency, **fL40,0.** The same meaning of the term *"centered"* as defined above with respect to **[fL1]** applies *mutatis mutandis* to **[fL0].** The number **m** of excitation bursts is an integer greater than 0. The excitation step brings to an excited state the excitable atoms **(A0)** which are not affected substantially by the hadron beam and which were therefore not brought to a saturated state by the saturating electromagnetic field, **B1-sat.** Preferably, the A0-excitation step rotates the net polarization vector by about 90°, as illustrated in the graph of Figure 8(b). During the A0-excitation step, the spins of the excitable atoms **A0** are preferably brought into phase. Magnetic resonance data can be collected and images generated as explained above based on the RF-signals emitted by the excitable atoms A0 upon relaxation, based on either or both T1 and T2 relaxation times.

The period of time, **Δts-e,** separating the A1-saturation step from the A0-excitation step (i.e., separating the n^{th} of the **B1-sat** bursts, from the first of the **p B1-exc** bursts) is critical for the visualization of the hadron beam path. In one embodiment, the period of time, **Δts-e,** can be very short, as short as zero, such that when the excitation step starts, the irradiated excitable atoms **A1** which are in or close to a saturated state cannot react to the **p** bursts of **B1**-**exc**. In this embodiment the period of time, **Δts-e,** is preferably not longer than half the longitudinal relaxation time, T1, of the excitable atoms **A0,** more preferably not longer than **T1** / 3, even more preferably not longer than **T1** / 4, which is considered as short enough for the irradiated excitable atoms **A1** to be close enough to a saturated state to not respond substantially to the A0-excitation step. In this case, the period of time, **Δts-e,** is in practice preferably not more than 100 ms / T, more preferably not more than 70 ms / T, even more preferably not more than 50 ms / T.

In an alternative embodiment, wherein the A1-saturation step includes no phasing of the spins, resulting in a X- and Y-components, **Mx,y,** of the net polarization substantially equal to zero, the period of time, **Δts-e,** is preferably comprised within ± 20% of the time, **tM0,** at which the Z-component, **Mz,** of the net polarization vector, **M,** of the irradiated excitable atoms **A1**, is zero, so that **Mz** is too small for contributing to the RF-signals collected by the antennas. More preferably, the period of time, **Δts-e,** is comprised between 0.8 **tM0** and 1.05 **tM0**. In practice, it is preferred that the period of time, **Δts-e,** is not more than 50 ms / T. Using T2 weighed imaging would therefore not detect the relaxations of the irradiated excitable atoms **A1**.

A hadron therapy device is provided suitable for directing a hadron beam along a beam path intersecting said target body in a number, **N,** of hadron pulses of pulse periods, Pbi, wherein, **N** is an integer greater than 0. The hadron beam has an initial energy, **E0,** previously determined during the establishment of a treatment plan for reaching the target tissue **40** at the level of an iso-energy layer, **Vti,** comprising target spots **40si,j** (cf. Figure 4(b)). In order to visualize the hadron beam traversing the target tissue in a MRI image, the hadron beam pulses emitted by the hadron therapy device must be synchronized in a specific manner with the excitement step, MRe, of the MRI described above. A hyposignal representative of the hadron beam path can be visualized if a significant concentration of irradiated excitable atoms A1 is present during the A1-saturation step. This can be achieved if the N hadron pulses overlap with at least 50% of the **n** bursts of the saturating electromagnetic field, **B1-sat** during the A1-saturation step. Without wishing to be bound by any theory, this synchronization is required because the magnetic susceptibilities of the irradiated excitable atoms **A1** return rapidly to their original values after interruption of the hadron beam. It is estimated that the irradiated excitable atoms **A1** return to their original state **A0** in the order of µs after interruption of the hadron beam.

As illustrated in Figure 9(c), the overlap needs not be perfect. A hadron pulse **PB1** can be shorter than an A1-saturation burst **Ps1** and entirely or partly comprised in said burst. Several consecutive short hadron pulses can be comprised within an A1-saturation burst. Alternatively, a longer hadron pulse **PB2** can overlap with several A1-saturation bursts **Psi.** All is required is that there is an overlap of at least 50%, preferably at least 70%, more preferably at least 80%, and even more preferably at least 90% and most preferably 100% between the **N** hadron pulses and the **n** A1-saturation bursts.

A hadron pulse does not consist of hadrons flowing continuously during the whole period **PBi** of the hadron pulse. A hadron pulse is actually formed by consecutive trains of hadrons. In the present invention, consecutive trains of hadrons separated from one another by a period of not more than 1.5 ms are considered to form a single hadron pulse. Inversely, if two trains of hadrons are separated by a period of more than 1.5 ms, they are considered as belonging to two separate hadron pulses. For example, a synchro-cyclotron emitting a 10 µs-hadron train every 1 ms during 10 ms is considered herein as forming a single hadron pulse of period **PBi =** 10 ms. Typically, a hadron pulse can have a period, **PBi,** comprised between 10 µs and 30 ms, depending on the type of hadron source used. In one example, the hadron beam pulse period, **PBi,** is preferably comprised between 1 ms and 10 ms. In another embodiment, it is preferably comprised between 5 and 20 ms. As discussed above with respect to Figure 4(c), two consecutive hadron pulses can be separated from one another by a period, **ΔPBi,** comprised between 1 and 20 ms, preferably between 2 and 10 ms.

Figure 9(d) illustrates schematically the spins of the excitable atoms A0 and A1 as the excitation sequence proceeds. During the A1-saturation step carried out at a frequency range, **[fL1],** excluding the Larmor frequency, **fLm0,** of the excitable atoms **A0,** unaffected by the hadron beam, the net polarization vectors of the excitable atoms A0 remains unaffected and is parallel to B0. The irradiated excitable atoms A1, on the other hand, are excited to saturation, in that the Z-component, Mz, of their magnetic moment is rotated by an angle comprised between 100 and 180°, preferably comprised between 160 and 180°.

A time, **Δts-e,** after the end of the saturation step, the excitation step is started. In Figure 9(d), the time, **Δts-e,** corresponds to about the time, **tM0**, required for the Z-component, **Mz,** of the net polarization vector of the irradiated excitable atoms **A1** to become zero. As discussed above, a time, **Δts-e,** not longer than **T1** / 2, wherein **T1** is the longitudinal relaxation time of the excitable atoms, **A0,** can alternatively be selected (cf. Figure 8(a)). The frequency range, **[fL0],** may or not include the Larmor frequency, **fLm, 1,** of the irradiated excitable atoms **A1**, and the bandwidth of **[fLm0],** can be freely selected based on other requirements, such as the desired thickness, **Δxi**, of the imaging layer, **Vpi.** The spins of the excitable atoms A0 are excited and rotated by an angle of e.g., 90° (cf. Figure 8(b) and 9(d)).The irradiated excitable atoms having a **Mz** value of about zero cannot be excited by the magnetic field **B1-exc** and emit substantially no RF signal receivable by the antennas **2a.** In case the time, **Δts-e,** is shorter, the spins of the irradiated excitable atoms **A1** are still saturated and cannot react to the excitation step **B1-exc.** The MR data thus acquired yields an image wherein the zones comprising irradiated excitable atoms **A1** are visible as a hyposignal compared with the zones comprising non-irradiated excitable atoms **A0,** as discussed below with reference to Figure 10. In order to capture the whole of a hadron beam path, it is preferred that the first direction, **X1,** defining the thickness, **Δx1,** of an imaging layer, **Vpi,** be normal to the hadron beam **1h** as shown e.g., in Figures 6(a)&(b), and 10, so that the hadron beam is comprised in a single imaging layer.

Figure 10 illustrates (a) the shift, **ΔfL40**, between the Larmor frequencies of excitable atoms **A0** and irradiated excitable atoms **A1** located in a target tissue **40** exposed to a main magnetic field, **B0**. The target tissue **40** is typically a tumour composed of cancerous cells. Figure 10(b) illustrates schematically an image of the tissues traversed by a hadron beam **1h** represented by a thick dashed line reaching a target spot **40s** located in the target tissue **40.** The hadron beam **1h** crosses a number of healthy tissues **41-43** before reaching the target tissue **40** and the target spot **40s.** The tissue **41** can typically be the skin of a patient. The thin dotted line represents an irradiated volume surrounding the hadron beam **1h** containing the irradiated atoms **A1** which magnetic susceptibility is being modified by the passage of the hadron beam and are characterized by a Larmor frequency, **fLm1,** with **m** = **40** to **43.** Outside said irradiated volume the magnetic susceptibility of the excitable atoms **A0** is not affected significantly by the hadron beam and their Larmor frequency is **fLm0,** with **m** = **40-43.** Tissue **44** is a healthy tissue, possibly a vital tissue, located downstream of the target tissue **40,** and should not be reached by the hadron beam.

Figure 10(c) shows the Energy loss curve of the hadron beam **1h** as it travels across the tissues until reaching the target spot in the target tissue. The hadron beam has an initial energy, **E0,** (i.e., before reaching the first tissue **41** along its beam path) which had been determined previously during the establishment of a treatment plan. If the treatment plan was performed accurately and if the relative positions and morphologies of the tissues **40 -43** traversed by the hadron beam had not had changed since the establishment of the treatment plan, the Bragg peak of a hadron beam of initial energy, **E0,** should fall at the position of the target spot **40s.** This optimal situation is illustrated in Figure 10(c).

As discussed above, it is quite possible, however, that the sizes and positions of the tissues traversed by a hadron beam change between the day, **t0**, a treatment plan had been established and the day, **t1,** of a hadron therapy session. Figure 10(e) illustrates a case wherein the tissues **42** and **43** located upstream from the target tissue 40 have shrunk between **t0** and **t1**. Tissues **42** and **43** could be fat and muscles which can easily shrink during an illness. Consequently, the target tissue has moved closer to the upstream boundary of the treated anatomy and the distance the hadron beam must travel across tissues until the actual position of the target spot **40s(t1)** has decreased accordingly. Irradiation of the tissues with a hadron beam of initial energy, **E0,** reaches beyond the actual position of the target spot. As illustrated in Figure 1, the identification of such mismatch between the planned position **P0** and the actual position **P1** in existing methods would lead to the interruption of the treatment session and to the establishment of a new treatment plan, wasting precious time and resources.

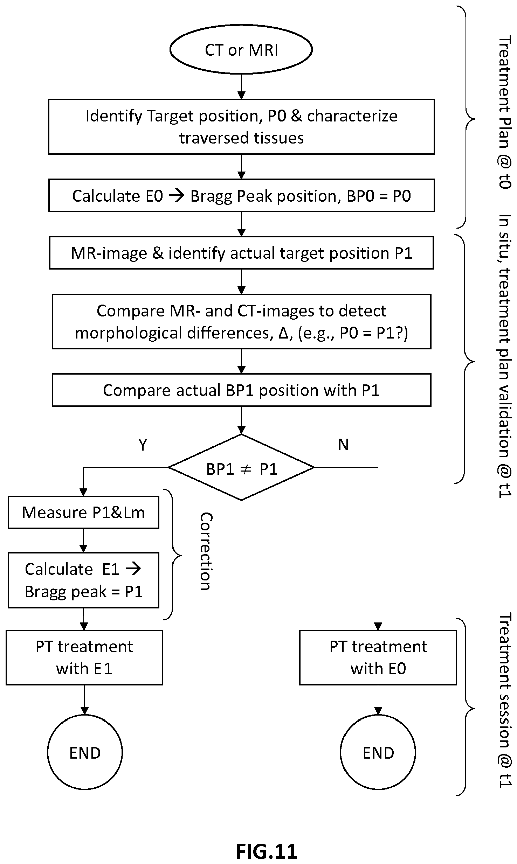

As discussed supra in relation with Figure 1 (cf. "∃Δ ?" → Y), absent the actual positon of the Bragg peak of a hadron beam of initial energy, **E0,** the day **t1** of a hadron therapy session, in case the MR images revealed any change of morphology or position of the tissues surrounding and including the target tissue since the day **t0** of the treatment plan, the hadron therapy session had to be stopped and a new treatment plan established. As illustrated in Figure 11, by visualizing the hadron beam obtained by the present invention, it is possible to identify a mismatch between the actual position of the Bragg peak, **BP1**, and the actual position, **P1**, of the target spot (even if **P1** = **P0**). The major advantage of the present invention is that it is possible to correct *in situ* the initial energy, **E1,** required for the Bragg peak to fall on the actual position **P1** of the target spot. The correction involves the measurement of the actual position **P1** of the target spot, and of the thicknesses, **Lm,** with **m** = 40-43, of the various tissues the hadron beam must traverse to reach the target spot **40s,** to determine the actual distance the hadron beam must travel through the tissues to reach the target spot. Through the determination of the corresponding WEPL's discussed above, it is possible to calculate the initial energy, **E1,** required for the position of the Bragg peak of the hadron beam to overlap with the actual position, **P1,** of the target spot **40s.** The treatment can thus proceed the same day with the corrected initial energy, **E1.** The advantage this represents over the prior art is enormous in terms both economical and of the health of the patients.

The doses deposited onto the tissues for visualizing the hadron beam path must be low, because in case of a change of morphology of the tissues, a full therapeutic dose reaching healthy tissues would be extremely detrimental to the health of a patient. For this reason, the hadron doses deposited for the visualization of the hadron beam are substantially lower than the therapeutic doses required for treating the target tissue and have substantially no therapeutic effects. As discussed with respect to Figure 4(c), this can be achieved either by irradiating few target spots, e.g., irradiating 1 to 40% of the target spots of an iso-energy layer, **Vti,** preferably 5 to 30%, more preferably 10 to 20%. Alternatively or concomitantly, target spots can be irradiated with a hadron beam having an intensity substantially lower than prescribed by the treatment plan. Finally, the irradiation time, **ti,** can also be reduced considerably, to the minimum required for acquiring an MR-image. In these conditions, the validation of the treatment plan is safe for the patient, even if a correction of the initial energy is required. In practice, it is preferred to irradiate only a selection of the target spots **40si,j** of the target tissue to yield the relative positions of the Bragg peak, **BP1,** and the corresponding target spot, 40s, to calculate the initial energy, **E1,** which can be used during the treatment session to treat all the target spots **40si,j** of an iso-energy volume, **Vti.** The initial energies required for treating target spots, **40(i+1),j,** ... in subsequent iso-energy volumes, **Vt(i+1),** ..., can either be extrapolated from the initial energy, **E1,** determined for the iso-energy volume, **Vti,** or, alternatively or additionally, a selection of target spots **40(i+1),j,** ... of the subsequent energy volumes, **Vt(i+1),** ... can be tested as described above.

The present invention also concerns a medical apparatus for carrying out the foregoing method of visualizing a hadron beam together with the target tissue it must irradiate. The medical apparatus of the present invention comprises the following components:
(a) a hadron source adapted for irradiating a target tissue **40** with a hadron beam **1h** having a beam energy, **Ek,** with **k =** 0 or 1, along a beam path in a number, **N,** of hadron pulses;
(b) a magnetic resonance imaging device (MRI) for the acquisition of magnetic resonance (MR) images within an imaging volume, **Vp,** including the target tissue **40,** as the target tissue is being irradiated,
(c) a controller configured for controlling the hadron source and for acquiring magnetic resonance images by implementing the following steps:
   - creating a main magnetic field, **B0**, in the imaging volume, **Vp,** including the target tissue **40,**
   - creating **n** bursts of a **saturating electromagnetic field, B1-sat,** wherein **n** is an integer greater than 0, which oscillates at a frequency range, **[fL1],** of band width, **b1** < **2·ΔfL40,** and centred on the Larmor irradiated frequency, **fL40,1,** and excluding the Larmor rest frequency, **fL40,0,** wherein
      o **fL40,0** is the rest Larmor frequency of excitable atoms **A0** present in the target tissue **40;**
      o **fL40,1** is the irradiated Larmor frequency of **irradiated excitable atoms A1** defined as the excitable atoms **A0** present in the same target tissue, and exposed to the effects of a hadron beam of initial energy, **E0,** traversing said target tissue positioned in the same magnetic field, **B0,** and wherein
      o **ΔfL40** = **|fL40,1 - fL40,0|;**
   - after a time, **Δts-e,** following the nth burst **B1-sat,** creating p bursts of an exciting electromagnetic field, **B1-exc,** oscillating at a frequency range, **[fL0],** centred on the Larmor rest frequency, **fL40,0,** wherein m is an integer greater than 0;
   - directing a hadron beam having the initial energy, **E0,** along a beam path intersecting said target tissue in a number, **N,** of hadron pulses of pulse periods, **Pbi ,** wherein, **N** is an integer greater than 0;
(d) a display for representing the target tissue from the magnetic resonance data acquired by the MRI within the imaging volume, **Vp,** and for visualizing the beam path in the target tissue as a hyposignal **1p,** weaker than the signal generated by the excitable atoms **A0** which are not exposed significantly to the effects of the hadron beam,
   **characterized in that,** said controller is further configured for synchronizing the **N** hadron pulses to overlap with at least 50% of the **n** bursts of the saturating electromagnetic field, **B1-sat.**

## Claims

1. Method for visualizing a hadron beam traversing an organic body, said method comprising:
(a) determining:
• the Larmor rest frequency, fLm,0, of an excitable atom (A0) present in a target tissue m of a subject of interest, wherein m=40, corresponding to a target tissue, and exposed to a uniform magnetic field, B0, and
• the Larmor irradiated frequency, fLm,1, of irradiated excitable atoms (A1) defined as the excitable atoms (A0) present in the same tissue m, and exposed to the effects of a hadron beam of initial energy, Ek, with k = 0 or 1, traversing said target tissue positioned in the same magnetic field, B0, and
(b) calculating the frequency shift, ΔfLm=|fLm,1 - fLm,0|, in the target tissue, with m=40;
(c) providing a magnetic resonance imaging device (MRI) for acquiring magnetic resonance data within an imaging volume, Vp, including the target tissue, positioned in the uniform main magnetic field, B0;
(d) providing a hadron source adapted for directing a hadron beam having an initial energy, E0, along a beam path, Xp, intersecting the target tissue in the imaging volume;
(e) acquiring magnetic resonance data from the imaging volume, by at least an excitation step (MRe), the excitation step comprising:
• an A1-saturation step, comprising creating n bursts of a saturating electromagnetic field, B1-sat, wherein n is an integer greater than 0, which oscillates at a frequency range, [fL1], of band width, b1 < 2·ΔfL40, and centred on the Larmor irradiated frequency, fL40,1, and excluding the Larmor rest frequency, fL40,0, such as to bring the nuclei of the irradiated excitable atoms (A1) to a saturated state wherein a net polarization vector of the spins is reversed at an angle comprised between 100 and 180° with respect to the net polarization vector of the spins of said nuclei at rest (i.e., absent B1-sat) and, after a time, Δts-e, following the n^{th} burst B1-sat:
• an AO-excitement step comprising creating m bursts of an exciting electromagnetic field, B1-exc, oscillating at a frequency range, [fL0], centred on the Larmor rest frequency, fL40,0, wherein m is an integer greater than 0, such as to bring to an excited state the excitable atoms (AO) which are not affected by the hadron beam and which were therefore not brought to a saturated state by the saturating electromagnetic field, B1-sat,
(f) directing a hadron beam having the initial energy, E0, along a beam path intersecting said target body in a number, N, of hadron pulses of pulse periods, Pbi, wherein, N is an integer greater than 0;
(g) representing on a display the organic body from the magnetic resonance data acquired by the MRI within the imaging volume, Vp,
(h) on the display, visualizing the beam path in the target tissue as a hyposignal (1p), weaker than the signal generated by the excitable atoms (A0) that are not exposed significantly to the effects of the hadron beam,
**characterized in that,** the N hadron pulses overlap with at least 50% of the n bursts of the saturating electromagnetic field, B1-sat during the A1-stauration step.

2. The method according to claim 1, wherein the N hadron pulses overlap with at least 70%, preferably at least 80%, more preferably at least 90% and most preferably 100% of the n bursts of the saturating electromagnetic field, B1-sat, and wherein the N hadron pulses are preferably in phase with the n bursts of the saturating electromagnetic field, B1-sat.

3. The method according to claim 1 or 2, wherein the N hadron pulses have a period, PBi, comprised between 10 µs and 30 ms, preferably between 1 and 10 ms or, alternatively, preferably between 5 and 20 ms, and two hadron pulses are preferably separated from one another by a period, ΔPBi, comprised between 1 and 20 ms.

4. The method according to anyone of the preceding claims, wherein the period of each of the n bursts of the saturating electromagnetic field, B1-sat, is comprised between 1 and 20 ms, preferably between 2 and 10 ms.

5. Method according to anyone of the preceding claims, wherein the target tissue (40) is a tumour exposed to a uniform magnetic field, B0, and traversed by a hadron beam of initial energy, E0, and wherein the frequency shift, ΔfLm, at the level of the Bragg peak of said hadron beam is comprised between 60 and 6000 Hz, preferably between 200 and 1200 Hz in a main magnetic field, B0 = 1.5 T, or wherein the relative frequency shift, ΔfLmr, ranges from 0.9 to 93 ppm, preferably from 3 to 16 ppm.

6. Method according to anyone of the preceding claims, wherein the time period, Δts-e, separating the last burst of the n saturating electromagnetic field, B1-sat, and the first burst of the p exciting electromagnetic field, B1-exc, is either:
• Not more than 50% of a longitudinal relaxation time, T1(A0), of the excitable atoms (A0) not affected substantially by the hadron beam, wherein Δts-e is preferably not more than 100 ms / T; or
• Within ±20% of the time, tM0, required for the longitudinal component, Mz, parallel to B0 of the net polarization vector of the irradiated excitable atoms (A1) to pass from the saturated state to a value of zero, and wherein Δts-e is preferably not more than 50 ms / T.

7. Method according to anyone of the preceding claims, wherein the n bursts of saturating electromagnetic field, B1-sat are created as adiabatic bursts.

8. Method according to anyone of the preceding claims, wherein the imaging volume, Vp, is controlled by creating a magnetic gradient along, one, two, or three of the first, second, and third directions, X1, X2, X3, to control a thickness of the imaging volume along said first, second, or third directions, X1, X2, X3.

9. Method according to anyone of the preceding claims, comprising the following steps:
(a) establishing at day, t0, a treatment plan and determining an initial energy, E0, of a hadron beam for depositing a given dose of hadrons to a target spot (40s),
(b) comparing on the display the morphology and thicknesses of the tissues traversed by a hadron beam of initial energy, E0, at day, t1 > t0, with the morphology and thicknesses of the same tissues as defined in the treatment plan, at day, t0,
(c) visualizing on the same display the actual position of the Bragg peak of the hadron beam,
(d) in case of mismatch between the actual position of the Bragg peak and of the target tissue (40s), correcting the initial energy, E1, of the hadron beam required for the Bragg peak to fall over the target spot.

10. A medical apparatus comprising the following components:
(a) a hadron source adapted for irradiating a target tissue (40) with a hadron beam (1h) having a beam energy, Ek, with k = 0 or 1, along a beam path in a number, N, of hadron pulses;
(b) a magnetic resonance imaging device (MRI) for the acquisition of magnetic resonance (MR) images within an imaging volume, Vp, including the target tissue (40), as the target tissue is being irradiated,
(c) a controller configured for controlling the hadron source and for acquiring magnetic resonance images by implementing the following steps:
• creating a main magnetic field, B0, in the imaging volume, Vp, including the target tissue (40),
• creating n bursts of a saturating electromagnetic field, B1-sat, wherein n is an integer greater than 0, which oscillates at a frequency range, [fL1], of band width, b1 < 2·ΔfL40, and centred on the Larmor irradiated frequency, fL40,1, and excluding the Larmor rest frequency, fL40,0, wherein
o fL40,0 is the rest Larmor frequency of excitable atoms (A0) present in the target tissue (40);
o fL40,1 is the irradiated Larmor frequency of irradiated excitable atoms (A1) defined as the excitable atoms (A0) present in the same target tissue, and exposed to the effects of a hadron beam of initial energy, E0, traversing said target tissue positioned in the same magnetic field, B0, and wherein
o ΔfL40 = |fL40,1 - fL40,0|;
• after a time, Δts-e, following the nth burst B1-sat, creating m bursts of an exciting electromagnetic field, B1-exc, oscillating at a frequency range, [fL0], centred on the Larmor rest frequency, fL40,0, wherein m is an integer greater than 0;
• directing a hadron beam having the initial energy, E0, along a beam path intersecting said target tissue in a number, N, of hadron pulses of pulse periods, Pbi , wherein, N is an integer greater than 0;
(d) a display for representing the target tissue from the magnetic resonance data acquired by the MRI within the imaging volume, Vp, and for visualizing the beam path in the target tissue as a hyposignal (1p), weaker than the signal generated by the excitable atoms (A0) which are not exposed significantly to the effects of the hadron beam,
**characterized in that,** said controller is further configured for synchronizing the N hadron pulses to overlap with at least 50% of the n bursts of the saturating electromagnetic field, B1-sat.
